# EUROPEAN PATENT SPECIFICATION

(11) **EP 2 017 789 B1**
(45) Date of publication and mention of the grant of the patent: **19.03.2014**
(21) Application number: 08007139.2
(22) Date of filing: 10.04.2008
(51) Int. Cl.: G06T 15/00

(54) **Projection image generation apparatus and program**
Projektionsbilderzeugungsvorrichtung und Programm
Appareil de génération d'image de projection et programme

(30) Priority: 11.04.2007 JP 2007103593
(43) Date of publication of application: 21.01.2009
(73) Proprietor: FUJIFILM Corporation, Minato-ku Tokyo (JP)
(72) Inventor: Masumoto, Jun, Chiba (JP); Miyamoto, Masaki, Tokyo (JP)
(74) Representative: Klunker . Schmitt-Nilson . Hirsch

(56) References cited:
- WO-A-2005/091226
- US-A1- 2006 104 545

## Description

### BACKGROUND OF THE INVENTION

### Field of the Invention

The present invention relates to a projection image generation apparatus and program for generating a projection image by projecting three-dimensional volume data.

### Description of the Related Art

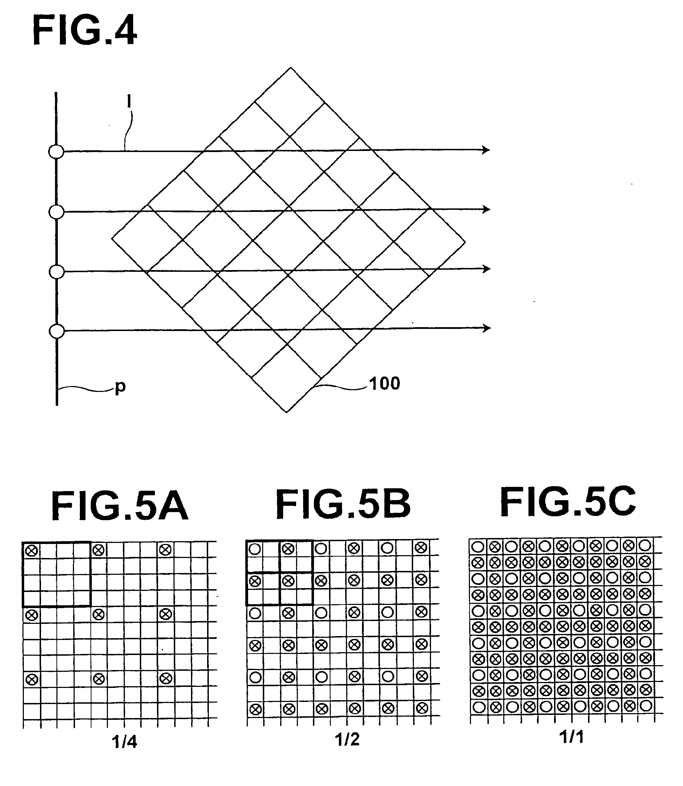

Conventionally, as a technique for visualizing and displaying volume data representing a three-dimensional object, volume rendering is well known. In volume rendering, there is a method called a ray casting method. In the ray casting method, an image is generated based on a projection plane on an observing side. The sizes of voxels of three-dimensional voxel data are determined by the number of pixels of tomographic images (slice images) that have been obtained by a CT apparatus or the like and intervals of imaging in the direction of the body axis of a subject. Therefore, if the number of pixels on the projection plane on the observing side is determined, the density of the voxels and that of the pixels are not always the same. Further, when an object is rotated, the intervals of voxels observed from the projection-plane side become irregular, as illustrated in Figure 4. Therefore, to obtain an image that looks natural, a ray of light is extended (casted or sent) from a pixel on the projection-plane side and data on the projection image is calculated from voxels through which the ray passes.

Such volume rendering is often used in medical practice to visualize a three-dimensional object, such as an internal organ, on a display. An image, in which only a blood vessel or an internal organ is projected, is generated to observe (examine) a diseased part of a patient. The image is generated by performing an operation of adding voxel data through which the ray has passed. Further, in many cases, images of the blood vessel or the internal organ are observed from different directions while the images are rotated. Therefore, it is desirable that the image is quickly switched to an image in each of the different directions and smoothly displayed.

However, the data amount of voxel data is enormously large and the load of calculation by the operation of addition is extremely high. Therefore, long time is required to switch the displayed images. Hence, various kinds of methods for displaying images at high speed have been proposed. Most of volume data includes a large amount of data within the space other than data of an organ which a user has interest in. However, data that is necessary for volume rendering is voxels corresponding to the external surface of each organ. Since the ray disappears at the external surface of each organ, it is not necessary to perform calculation for the portion that is deeper than the external surface of each organ. Therefore, a method has been proposed, in which when first, a user performs observation based on information about the depth while clicking a mouse, the volume data is divided into blocks, each having a size of 2x2x2. Then, a low-resolution rendering image is generated by using an average value of voxels included in each block of 2x2x2 and displayed. When the mouse is moved to a region of interest and the user releases the clicked state of the mouse, a high-resolution rendering image is generated by using the depth information and the original data and displayed at high speed (for example, Japanese Unexamined Patent Publication No. 2002-133441).

In WO 2005/091226 a similar technique is disclosed.

Further, as the volume rendering technique, a hybrid-type ray casting method is well known. In the hybrid-type ray casting method, object-order processing is performed. In the object-order processing, a depth map showing the distribution of distances between a projection plane and a three-dimensional object is generated whenever the projection plane is set. Then, image-order processing is performed. In the image-order processing, sampling is performed along each ray by using the generated depth map. Accordingly, the volume data is visualized. When the projection image is generated by using the hybrid-type ray casting method, hybrid-type volume rendering is performed on an octree generated from voxel data. Then, a division hierarchical level at which a calculation cost becomes the lowest is set by changing the division hierarchical level of the octree stepwise. Then, volume rendering is performed based on a change in the direction of the ray, in other words, a change in the projection plane, by utilizing octree data based on the set division hierarchical level. Accordingly, displayed images are smoothly changed (for example, Japanese Unexamined Patent Publication No. 2006-099422).

In each of the aforementioned methods, the load of calculation is reduced by changing the sizes of the voxels of the volume data. However, a long calculation time is required even for the processing of changing the sizes of the volume data, because the data amount of the volume data is large. Further, when an image that has a higher resolution is generated, calculation must be performed by using original volume data again.

### SUMMARY OF THE INVENTION

In view of the foregoing circumstances, it is an object of the present invention to provide a projection image generation apparatus and its program for smoothly displaying projection images by switching them at high speed based on an operation for rotating or moving the images.

A projection image generation apparatus according to the present invention is a projection image generation apparatus for projecting three-dimensional volume data that has been obtained by imaging a subject in such a manner that the projection direction or projection position thereof can be changed by an operation from the outside of the apparatus, the apparatus comprising:
a resolution setting means for setting the resolution of a projection image in a region of interest (attention) on a projection plane, onto which the three-dimensional volume data is projected, higher than that of the projection image on the projection plane other than the region of interest when the projection direction or the projection position has been changed by the operation;
a projection image generation means for generating a projection image by dividing, based on the set resolutions, the projection plane into small areas that have different sizes depending on whether an area of the projection plane to be divided is in the region of interest or not and by calculating the pixel value of each of the divided small areas from the voxel values of the three-dimensional volume data that is projected onto the respective small areas; and
a resolution changing means for repeatedly generating projection images in such a manner that the resolution of at least the projection image on the projection plane other than the region of interest is reset gradually to a higher value after the projection image generation means has finished calculation of the pixel values of the small areas, into which the projection plane has been divided.

Further, a program according to the present invention is a program for causing a computer of a projection image generation apparatus for projecting three-dimensional volume data that has been obtained by imaging a subject in such a manner that the projection direction or projection position thereof can be changed by an operation from the outside of the apparatus to function as:
a resolution setting means for setting the resolution of a projection image in a region of interest on a projection plane, onto which the three-dimensional volume data is projected, higher than that of the projection image on the projection plane other than the region of interest when the projection direction or the projection position has been changed by the operation;
a projection image generation means for generating a projection image by dividing, based on the set resolutions, the projection plane into small areas that have different sizes depending on whether an area of the projection plane to be divided is in the region of interest or not and by calculating the pixel value of each of the divided small areas from the voxel values of the three-dimensional volume data that is projected onto the respective small areas; and
a resolution changing means for repeatedly generating projection images in such a manner that the resolution of at least the projection image on the projection plane other than the region of interest is reset gradually to a higher value after the projection image generation means has finished calculation of the pixel values of the small areas, into which the projection plane has been divided.

The expression "dividing, based on the set resolutions, the projection plane into small areas that have different sizes depending on whether an area of the projection plane to be divided is in the region of interest or not" means that a part of the image in which the resolution on the projection plane is set to a high level is divided, based on the resolution, into small areas that have a relatively small size and that a part of the image in which the resolution on the projection plane is set to a low level is divided, based on the resolution, into small areas that have a relatively large size.

The expression "... has finished calculation of the pixel values of the small areas, into which the projection plane has been divided" means that the pixel values on the projection plane have been calculated at the set resolutions while neither the projection direction nor the projection position is changed by an operation from the outside.

It is desirable that the projection image generation means calculates the pixel value of each of the small areas from the voxel values of voxels of the three-dimensional volume data, through which a ray that has passed through the respective small areas and travels in the projection direction passes.

Further, it is desirable that the resolution changing image generation means repeatedly generates the projection images in such a manner that the set resolution is gradually increased to a higher value from the region of interest toward the outside of the projection plane.

According to the present invention, when the projection direction or the projection position of three-dimensional volume data is changed by an operation for rotating or moving a three-dimensional image, the resolution of a projection image in a region of interest on a projection plane is set higher than that of the projection image other than the region of interest. Then, the projection plane is divided into small areas based on the resolutions and a pixel value for each of the small areas is obtained to generate an image on the projection plane. Accordingly, it is possible to reduce a calculation amount for the low-resolution image portion. Since a projection image that has high resolution only within the region of interest is generated, it is possible to rotate or move the image at high speed by an operation from the outside of the apparatus. Further, if neither the projection direction nor the projection position is changed, projection images are repeatedly generated gradually at higher resolutions. Consequently, it becomes possible to observe the whole area of the projection image in detail.

Further, since the projection images are repeatedly generated in such a manner that the set resolution is increased gradually to a higher value from the region of interest toward the outside of the projection plane, it is possible to check an area in the vicinity of the point of interest at an early stage of observation.

### BRIEF DESCRIPTION OF THE DRAWINGS

Figure 1 is a schematic diagram illustrating the configuration of a projection image generation apparatus;
Figure 2 is a diagram for explaining division of a projection plane into small areas;
Figure 3 is a diagram illustrating an example of a projection image;
Figure 4 is a diagram for explaining ray casting;
Figure 5A is a diagram for explaining a method for calculating pixel values when the resolution is increased;
Figure 5B is a diagram for explaining the method for calculating pixel values when the resolution is increased;
Figure 5C is a diagram for explaining the method for calculating pixel values when the resolution is increased; and
Figure 6 is a flowchart for explaining the flow of processing by the projection image generation apparatus.

### DESCRIPTION OF THE PREFERRED EMBODIMENTS

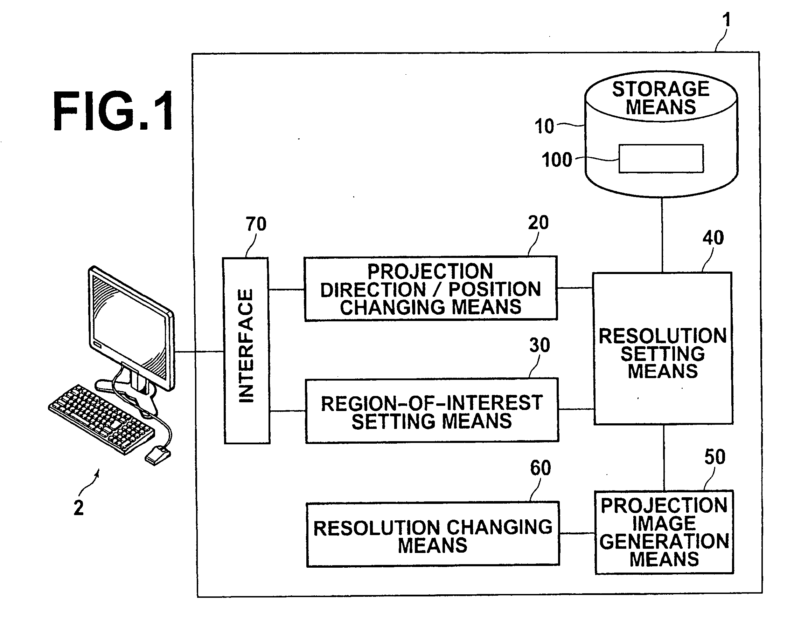

Hereinafter, embodiments of a projection image generation apparatus of the present invention will be described in detail with reference to drawings. Figure 1 is a schematic diagram illustrating the configuration of a projection image generation apparatus of the present invention. The configuration of a projection image generation apparatus 1, as illustrated in Figure 1, can be realized by causing a computer to execute a projection image generation processing program that has been installed in an auxiliary storage apparatus. The projection image generation processing program is stored in a recording medium, such as a CD-ROM, or distributed through a network, such as the Internet, and installed in the computer.

The projection image generation apparatus 1 includes a storage means 10, an interface 70, a projection direction/position changing means 20, a region-of-interest setting means 30, a resolution setting means 40, a projection image generation means 50 and a resolution changing means 60. The storage means 10 stores three-dimensional volume data 100, which constitutes a three-dimensional medical image that has been obtained by imaging a subject. The interface 70 receives operation information based on an operation for rotating or moving an image from an input apparatus 2. The projection direction/position changing means 20 changes, based on the input operation information, a projection direction or a projection position for projecting the volume data 100 onto a projection plane. The region-of-interest setting means 30 sets a region of interest in a projection image, onto which the volume data 100 has been projected. The resolution setting means 40 sets the resolution of a projection image within a region of interest on the projection plane, onto which the volume data 100 is projected, higher than that of the projection image on the projection plane other than the region of interest. The projection image generation means 50 generates a projection image by dividing, based on the set resolutions, the projection plane into small areas and by calculating the pixel value of each of the divided small areas from the voxel values of the volume data 100. The resolution changing means 60 repeatedly generates projection images in such a manner that the resolution of the projection image on the projection plane is reset (repeatedly set) gradually to a higher value after the projection image generation means 50 has finished calculation of all of the pixel values of the small areas.

The storage means 10 is a high-capacity storage apparatus, such as a hard disk. Three-dimensional volume data 100 is stored in the storage means.

The three-dimensional volume data 100 is represented by a set of voxels, which are formed by dividing a three-dimensional space into small cubes. The three-dimensional volume data 100 represents the density and the density distribution of an image within the three-dimensional space by using three-dimensional data array. Specifically, the volume data 100 is obtained by superposing, one on another, two-dimensional tomographic image data, which is sequentially obtained along a direction perpendicular to a tomographic plane of an object, which is a target of processing. The volume data 100 is generated by superposing, one on another, a plurality of tomographic images obtained by imaging using a CT apparatus, an MRI apparatus or the like. For example, if the volume data 100 is obtained by using the CT apparatus, data storing an absorption amount of X rays is obtained for each voxel. A single voxel value (In the CT apparatus, a value representing an absorption amount of X-rays) is provided for each voxel. Hereinafter, the picture-element (voxel) value of each voxel is referred to as a voxel value.

The interface 70 receives a signal generated by an operation using the input apparatus 2, such as a mouse and a tracking ball, as operation information from the input apparatus 2. At the input apparatus 2, the signal is generated by an operation for rotating or moving an image.

The projection direction/position changing means 20 changes, based on the operation information that has been received at the interface 70, a projection direction or a projection position for projecting the volume data.

The region-of-interest setting means 30 sets a region of interest in the projection image that is displayed on a monitor screen. For example, the region-of-interest setting means 30 makes a user specify a portion of the projection image that he/she wants to observe in detail by using a pointing device, such as a mouse. Then, the region-of-interest setting means 30 sets a rectangular area, the center of which is a point that has been determined (pointed or specified) by the user, as a region of interest.

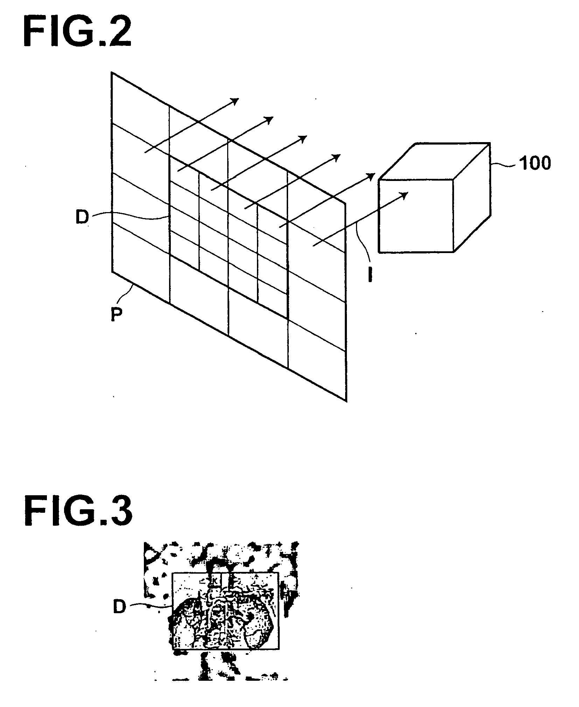

The resolution setting means 40 sets the resolution of a projection image in a region of interest on a projection plane higher than that of the projection image on the projection plane other than the region of interest whenever the projection direction or the projection position has been changed. For example, as illustrated in Figure 2, the resolutions are set in such a manner that the resolution within the region D of interest near the center of the projection plane P becomes four times as high as that of the other area of the projection plane P around the region D of interest.

The projection image generation means 50 divides, based on the resolutions that have been set by the resolution setting means 40, the projection plane P into small areas. As illustrated in Figure 2, a low-resolution area is divided into small areas that are relatively large. Each part of the projection plane P is divided into smaller small areas as the resolution of the part is higher. Further, the pixel value of each of the small areas is calculated by ray casting by using a volume rendering method and a projection image is generated.

As illustrated in Figure 4, in the ray casting method, a virtual projection plane P is arranged within a three-dimensional space. Then, a virtual ray that is called as a ray 1 is casted (sent or output) from each pixel on the projection plane P. Then, an image of virtual reflection light that has been reflected from the inside of the volume data 100 is formed. Accordingly, a projection image showing a three-dimensional structure within the volume data 100 is generated on the projection plane P. Specifically, simulation of light, in which the light is sent from the projection plane P and the light is reflected, attenuated or absorbed by the volume data 100 represented by voxel values, is performed. It is possible to render (draw) an object structure based on the volume data by using such a volume rendering method. If the volume data 100 represents the structure of a human body, in which tissues, such as bones and internal organs, are complexly combined, these tissues can be rendered separately from each other by adjusting the transmittance (adjusting the opacity). Specifically, a projection image, in which the user can observe only a region that he/she wants to examine, can be generated by increasing the opacity of each of voxels composing the region to be observed and by lowering the opacity in a region not to be observed. For example, if the opacity of the skin or the like of a subject is set to a low level, it is possible to observe each organ, such as blood vessels, bones and internal organs, by looking through the human body.

Further, the pixel value of each of the divided small areas is obtained by applying (stretching or extending) a single pixel value (projection value) that has been calculated from the voxel values of volume data through which a ray that travels in a projection direction from a point within the respective small areas has passed thereto. Accordingly, a projection image is generated by using the ray casting method. Figure 3 illustrates an example of a projection image that has been generated by setting the resolution of only the region D of interest at a high value.

When the projection image generation means 50 has finished calculation of the pixel values of all of the small areas on the projection plane P, the resolution changing means 60 resets the resolution to a higher value and generates a projection image again. The projection image is a higher-resolution image. Further, when the whole projection image that has the higher resolution is generated, the resolution changing means 60 increases the resolution again and generates a projection image again. The resolution changing means 60 increases the resolution until the resolution reaches the maximum resolution.

It is desirable that high resolution is set for the region D of interest and low resolution is set for the projection plane other than the region D of interest from the beginning and that the resolution is gradually increased from the inside of the region D of interest toward the outside of the projection plane P until the resolution of the whole projection plane P finally reaches the maximum resolution. If the resolution is increased from the region D of interest toward the surrounding thereof, it is possible to check the region of interest and the neighborhood thereof (the vicinity thereof), in which the user has interest (to which the user pays attention), at an early stage of observation.

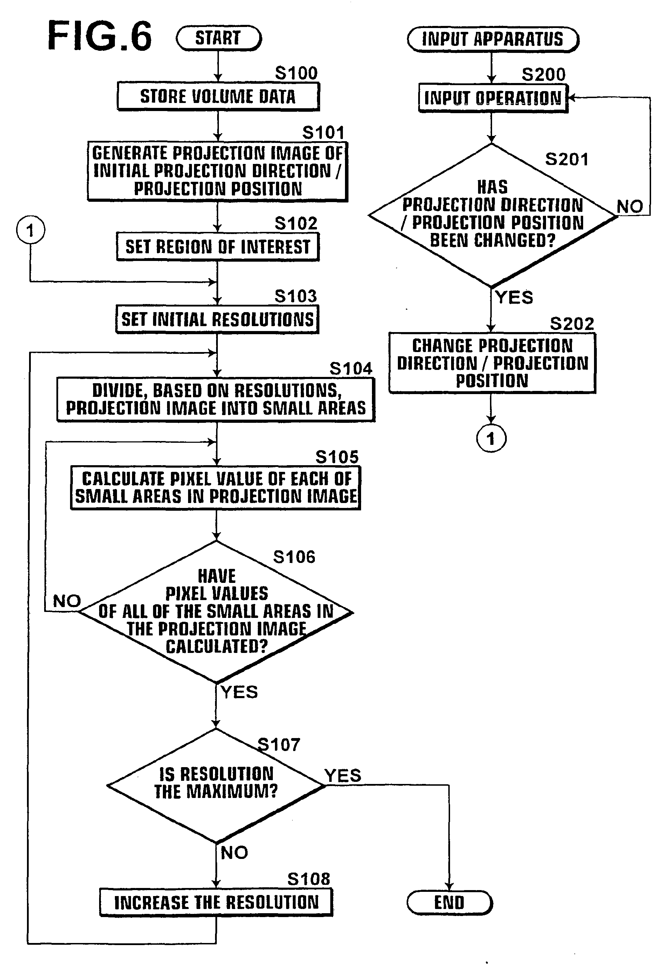

Next, with reference to the flowchart illustrated in Figure 6, an operation for diagnosing a patient while observing projection images using the projection image generation apparatus of the present invention and the flow of processing for generating the projection images based on the operation will be described.

A subject who is a target of diagnosis is imaged by using a CT apparatus or the like and tomographic images (slice images) are obtained. Then, volume data 100 is generated from the tomographic images and stored in a hard disk (storage means 10) (step S100). First, the volume data 100 is projected, based on an initial projection direction and an initial projection position that have been determined in advance, onto a projection plane P at the maximum resolution. The projection image is displayed on a monitor screen (display) (step S101). A user, such as a radiologist who is specialized in diagnosis using images, makes a diagnosis while observing the projection image. The user moves a pointer to the center of a part of the projection image that is displayed on the monitor by using a mouse or the like. The part of the projection image to which the pointer is moved is a part in which the user has interest. The radiologist observes the organ by performing an operation for rotating or moving the image of the organ with respect to the position of the pointer.

When the organ is displayed while being rotated or moved, it is necessary to repeatedly generate projection images that have different projection directions and different projection positions and to display the projection images on the monitor screen. However, if the whole image is displayed at high resolution, long time is required to generate the projection images and it becomes impossible to smoothly rotate or move the image of the organ. If the projection image is generated at low resolution and rotated or moved, the projection image may be rotated or moved at high speed. However, it is desirable that a region of the human body that may be diseased, which should be diagnosed, is displayed at high resolution even if the image of the organ is rotated or moved at high speed. Therefore, a projection image, in which only the region of interest has high resolution and the other part has low resolution, is generated.

First, the region-of-interest setting means 30 sets a rectangular area, the center of which is the position of the pointer determined by the user, as a region D of interest (step S102). The user performs an operation for rotating or moving the image by using the input apparatus 2, such as a mouse (step S200) . When the projection direction or the projection position is changed by the operation by the user (step S201), the projection direction/position changing means 20 changes the projection direction or the projection position (step S202). The resolution setting means 40 sets the resolutions to initial values whenever the projection direction or the projection position is changed. For example, within the region D of interest, the resolution is set to a value that is 1/2 of the maximum resolution with respect to each of the vertical direction and the horizontal direction. Further, in the area of the projection plane other than the region D of interest, the resolution is set to a value that is 1/4 of the maximum resolution with respect to each of the vertical direction and the horizontal direction. Accordingly, the resolution within the region D of interest is set to a high value and the resolution in the other area of the projection plane P, which excludes the region D of interest, is set to a low value (step S103).

The projection image generation means 50 divides, based on the set resolutions, the projection plane P into small areas (step S104). Then, a projection value that has been calculated from the voxel values of volume data through which a ray that travels in the projection direction from a point within each of the small areas has passed is applied (stretched or extended) to the entire area of the respective small areas. Accordingly, a projection image is generated. The projection image is displayed on the monitor screen (step S105). For example, if the resolution within the region D of interest is set to 1/2 of the maximum resolution with respect to each of the horizontal direction and the vertical direction, a small area within the region D of interest is composed of 2x2 pixels. The projection value of one of the 2x2 pixels is obtained, and the projection value is applied (stretched or extended) to pixels in the vicinity of the pixel. If the resolution on the projection plane P other than the region D of interest is set to 1/4 of the maximum resolution with respect to each of the horizontal direction and the vertical direction, a small area in the area other than the region D of interest is composed of 4x4 pixels. The projection value of one of the 4×4 pixels is obtained, and the projection value is applied (stretched or expanded) to pixels in the vicinity of the pixel.

Specifically, it is desirable that the pixel values of pixels between pixels, the projection values of which have been obtained, are obtained in such a manner that the pixel values of the pixels between the pixels gradually change. For example, as illustrated in Figure 5A, if the resolution is set to 1/4 of the maximum resolution with respect to each of the horizontal direction and the vertical direction, the projection value of every fourth pixel (pixels marked with x in Figure 5A) with respect to each of the horizontal direction and the vertical direction is obtained. The pixel values of pixels other than those marked with x are obtained by interpolation using the pixel values of pixels marked with x surrounding the respective pixels. In Figure 5A, if the pixel marked with x at the upper left corner is used as a base pixel, and if the right direction of the base pixel is + (positive) direction of the X-axis and the downward direction of the base pixel is - (negative) direction of the Y-axis, the positions of the pixels marked with x are represented as follows: (0, 0), (4, 0), (8, 0), ... (0, 4), (4, 4), (8, 4) ... (0, 8), (4, 8), (8, 8), ... . For example, the pixel value of pixel (1,1) is obtained by performing linear interpolation calculation using the pixel values of pixels (0, 0), (4, 0), (0, 4) and (4, 4), which are pixels in the same small area as pixel (1, 1) and in small areas adjacent to the small area to which pixel (1, 1) belongs. It is not necessary that the interpolation is performed by using the linear interpolation. Other interpolation methods may be used as long as the pixel values of pixels between pixels, the projection values of which have been obtained, gradually change. Alternatively, a nearest point interpolation method may be adopted. In the nearest point interpolation method, the same pixel value is used in the entire area of each of the small areas.

The projection image generation means 50 obtains the pixel values of all of the small areas on the projection plane P and the whole projection image is displayed on the monitor screen (step S106). Then, the resolution changing means 60 slightly increases the resolution (step S108). Further, the projection image generation means 50 generates a projection image again (steps S104 through S106). In this manner, whenever the whole projection image is displayed, the resolution of the projection image is gradually increased. Further, the resolution is gradually increased from the region D of interest toward the outside of the projection plane. Finally, the whole projection image is displayed at the maximum resolution (step S107).

Further, as illustrated in Figures 5A through 5C, when the resolution is increased, if the resolution is set to 1/4 of the maximum resolution with respect to each of the horizontal direction and the vertical direction, the projection value of every fourth pixel (pixels marked with x in Figure 5A) with respect to each of the horizontal direction and the vertical direction is obtained. Then, the obtained projection value is applied (stretched or extended) to 4x4 pixels that are present in the vicinity of the pixel, the projection value of which has been obtained, and a projection image is generated. If the resolution is increased from 1/4 of the maximum resolution with respect to each of the horizontal direction and the vertical direction to 1/2 of the maximum resolution with respect to each of the horizontal direction and the vertical direction, the projection values of pixels (pixels marked with x in Figure 5B) between the pixels, the projection values of which have been obtained at the resolution of 1/4 of the maximum resolution, are obtained. Then, the obtained projection values are applied to 2x2 pixels in the vicinity of the respective pixels, the projection values of which have been obtained. Accordingly, a projection image is generated. Further, the projection values of pixels (pixels marked with × in Figure 5C) between the pixels are obtained. Then, the projection values of all of the pixels are obtained and a projection image is generated.

When the user performs an operation again (step S200) and the projection direction or the projection position is changed (step S201), the projection direction/position changing means 20 changes the projection direction or the projection position (step S202). Then, the resolution setting means 40 returns the resolutions to initial values (1/2 of the maximum resolution with respect to each of the horizontal direction and the vertical direction for the region D of interest and 1/4 of the maximum resolution with respect to each of the horizontal direction and the vertical direction for the projection plane excluding the region D of interest) (step S103). Then, the projection image generation means 50 generates a projection image of a new projection direction (or a new projection position) (steps S104 through S106) . The resolution changing means 60 increases the resolution from the region D of interest toward the outside of the projection plane as long as neither the projection direction nor the projection position is changed (steps S107 and S108) .

As described above in detail, as long as neither the projection direction nor the projection position is changed, the resolution of the projection image is gradually increased. However, when the projection direction or the projection position is changed, the resolution is returned to the initial value. Then, a projection image is generated in such a manner that only the region of interest has high resolution and an area other than the region of interest has low resolution. Therefore, it is possible to smoothly rotate the image as soon as an instruction is given by the user.

In the above description, a case in which the region D of interest is pointed by a pointer or the like has been described. Alternatively, a region in the vicinity of the center of the projection image that is displayed on the monitor screen may be set as a region of interest.

Further, in the above description, a rectangular area around a point pointed by the pointer is used as a region of interest. Alternatively, a circle within a certain radius from the point that has been determined by the user may be used as a region of interest. Alternatively, the display may be divided in the vertical direction. Then, an area in the vicinity of the center of the display that is approximately 50% of the whole area may be displayed at high resolution and areas on the both sides may be displayed at low resolution. Further, if a region of interest is present in volume data, a cube that encloses (surrounds) the region may be obtained in advance. Then, a region projected onto the display may be obtained and used as a region of interest. The region of interest may be displayed at high resolution and the other area may be displayed at low resolution.

As described above in detail, if only the region of interest is displayed at high resolution and the other area of the projection image is displayed at low resolution, it is possible to smoothly rotate or move the image based on an operation by the user. Further, when the user does not perform an operation, the resolution is gradually increased. Therefore, it is possible to check the whole area in detail.

## Claims

1. A projection image generation apparatus for projecting three-dimensional volume data (100) that has been obtained by imaging a subject in such a manner that the projection direction or projection position thereof can be changed by an operation from the outside of the apparatus, the apparatus comprising:
a resolution setting means (40) for setting the resolution of a projection image in a region of interest on a projection plane, onto which the three-dimensional volume data (100) is projected, higher than that of the projection image on the projection plane other than the region of interest when the projection direction or the projection position has been changed by the operation;
a projection image generation means (50) for generating a projection image by dividing, based on the set resolutions, the projection plane into small areas that have different sizes depending on whether an area of the projection plane to be divided is in the region of interest or not and by calculating the pixel value of each of the divided small areas from the voxel values of the three-dimensional volume data (100) that is projected onto the respective small areas; and
a resolution changing means (60) **characterized in that** said resolution changing means is adapted for repeatedly generating projection images in such a manner that the resolution of at least the projection image on the projection plane other than the region of interest is reset gradually to a higher value after the projection image generation means (50) has finished calculation of the pixel values of the small areas, into which the projection plane has been divided.

2. A projection image generation apparatus, as defined in Claim 1, **characterized in that** the projection image generation means (50) calculates the pixel value of each of the small areas from the voxel values of voxels of the three-dimensional volume data (100), through which a ray that has passed through the respective small areas and travels in the projection direction passes.

3. A projection image generation apparatus, as defined in Claim 1 or 2, **characterized in that** the resolution changing means (60) repeatedly generates the projection images in such a manner that the set resolution is gradually increased to a higher value from the region of interest toward the outside of the projection plane.

4. A program for causing a computer of a projection image generation apparatus for projecting three-dimensional volume data (100) that has been obtained by imaging a subject in such a manner that the projection direction or projection position thereof can be changed by an operation from the outside of the apparatus to function as:
a resolution setting means (40) for setting the resolution of a projection image in a region of interest on a projection plane, onto which the three-dimensional volume data (100) is projected, higher than that of the projection image on the projection plane other than the region of interest when the projection direction or the projection position has been changed by the operation;
a projection image generation means (50) for generating a projection image by dividing, based on the set resolutions, the projection plane into small areas that have different sizes depending on whether an area of the projection plane to be divided is in the region of interest or not and by calculating the pixel value of each of the divided small areas from the voxel values of the three-dimensional volume data (100) that is projected onto the respective small areas; and
a resolution changing means (60) **characterized in that** said resolution changing means is adapted for repeatedly generating projection images in such a manner that the resolution of at least the projection image on the projection plane other than the region of interest is reset gradually to a higher value after the projection image generation means (50) has finished calculation of the pixel values of the small areas, into which the projection plane has been divided.

## Patentansprüche

1. Projektionsbild-Erzeugungsvorrichtung zum Projizieren dreidimensionaler Volumendaten (100), die gewonnen wurden durch Abbilden eines Subjekts in der Weise, dass seine Projektionsrichtung oder Projektionsstelle durch Betätigen von außerhalb der Vorrichtung geändert werden kann, umfassend:
eine Auflösungs-Einstelleinrichtung (40) zum Einstellen der Auflösung eines Projektionsbilds in einer interessierenden Zone einer Projektionsebene, auf die die dreidimensionalen Volumendaten (100) projiziert werden, auf einen höheren Wert als dem des Projektionsbilds auf die Projektionsebene verschieden von der interessierenden Zone, wenn die Projektionsrichtung oder die Projektionsstelle durch die Betätigung geändert wurde;
eine Projektionsbild-Erzeugungseinrichtung (50) zum Erzeugen eines Projektionsbilds, indem basierend auf den eingestellten Auflösungen die Projektionsebene aufgeteilt wird in kleine Bereiche mit unterschiedlichen Größen abhängig davon, ob ein Bereich der zu unterteilenden Projektionsebene die interessierende Zone ist oder nicht, und durch Berechnen des Pixelwerts jedes der abgeteilten kleinen Bereiche aus den Voxelwerten der dreidimensionalen Volumendaten (100), die auf die jeweiligen kleinen Bereiche projiziert werden; und
eine Auflösungs-Änderungseinrichtung (60), **dadurch gekennzeichnet, dass** die Auflösungs-Änderungseinrichtung dazu ausgebildet ist, wiederholt Projektionsbilder in der Weise zu erzeugen, dass die Auflösung mindestens des Projektionsbilds auf die von der interessierenden Zone verschiedene Projektionsebene allmählich auf einen höheren Wert neu-eingestellt wird, nachdem die Projektionsbild-Erzeugungseinrichtung (50) die Berechnung der Pixelwerte der kleinen Bereiche beendet hat, in die die Projektionsebene unterteilt wurde.

2. Projektionsbild-Erzeugungsvorrichtung nach Anspruch 1, **dadurch gekennzeichnet, dass** die Projektionsbild-Erzeugungseinrichtung (50) den Pixelwert jedes der kleinen Bereiche berechnet aus den Voxelwerten von Voxeln der dreidimensionalen Volumendaten (100), durch die ein Strahl verläuft, der durch die jeweiligen kleinen Bereiche hindurchgegangen ist und in die Projektionsrichtung läuft.

3. Projektionsbild-Erzeugungseinrichtung nach Anspruch 1 oder 2, **dadurch gekennzeichnet, dass** die Auflösungs-Änderungseinrichtung (60) wiederholt die Projektionsbilder in der Weise erzeugt, dass die eingestellte Auflösung ausgehend von der interessierenden Zone in Richtung der Außenseite der Projektionsebene allmählich auf einen höheren Wert gesteigert wird.

4. Programm zum Veranlassen eines Computers einer Projektionsbild-Erzeugungsvorrichtung zum Projizieren dreidimensionaler Volumendaten (100), die erhalten werden durch Abbilden eines Subjekts in der Weise, dass seine Projektionsrichtung oder Projektionsstelle durch Betätigung von außerhalb der Vorrichtung geändert werden kann, um zu fungieren als:
eine Auflösungs-Einstelleinrichtung (40) zum Einstellen der Auflösung eines Projektionsbilds in einer interessierenden Zone einer Projektionsebene, auf die die dreidimensionalen Volumendaten (100) projiziert werden, auf einen höheren Wert als dem des Projektionsbilds auf die Projektionsebene verschieden von der interessierenden Zone, wenn die Projektionsrichtung oder die Projektionsstelle durch die Betätigung geändert wurde;
eine Projektionsbild-Erzeugungseinrichtung (50) zum Erzeugen eines Projektionsbilds, indem basierend auf den eingestellten Auflösungen die Projektionsebene aufgeteilt wird in kleine Bereiche mit unterschiedlichen Größen abhängig davon, ob ein Bereich der zu unterteilenden Projektionsebene die interessierende Zone ist oder nicht, und durch Berechnen des Pixelwerts jedes der abgeteilten kleinen Bereiche aus den Voxelwerten der dreidimensionalen Volumendaten (100), die auf die jeweiligen kleinen Bereiche projiziert werden; und
eine Auflösungs-Änderungseinrichtung (60), **dadurch gekennzeichnet, dass** die Auflösungs-Änderungseinrichtung dazu ausgebildet ist, wiederholt Projektionsbilder in der Weise zu erzeugen, dass die Auflösung mindestens des Projektionsbilds auf die von der interessierenden Zone verschiedene Projektionsebene allmählich auf einen höheren Wert neu-eingestellt wird, nachdem die Projektionsbild-Erzeugungseinrichtung (50) die Berechnung der Pixelwerte der kleinen Bereiche beendet hat, in die die Projektionsebene unterteilt wurde.

## Revendications

1. Appareil de génération d'image de projection pour projeter des données de volume tridimensionnelles (100) qui ont été obtenues en imageant un sujet de telle sorte que la direction de projection ou sa position de projection puisse être changée par une opération depuis l'extérieur de l'appareil, l'appareil comprenant :
un moyen de réglage de résolution (40) pour régler la résolution d'une image de projection dans une région d'intérêt sur un plan de projection, sur lequel les données de volume tridimensionnelles (100) sont projetées, supérieure à celle de l'image de projection sur le plan de projection autre que la région d'intérêt lorsque la direction de projection ou la position de projection a été changée par l'opération ;
un moyen de génération d'image de projection (50) pour générer une image de projection en divisant, sur la base des résolutions réglées, le plan de projection en de petites zones qui ont des tailles différentes en fonction de si une zone du plan de projection à diviser est dans la région d'intérêt ou non et en calculant la valeur de pixel de chacune des petites zones divisées à partir des valeurs de voxel des données de volume tridimensionnelles (100) qui sont projetées sur les petites zones respectives ; et
un moyen de changement de résolution (60) **caractérisé en ce que** ledit moyen de changement de résolution est adapté pour générer de façon répétée des images de projection de telle sorte que la résolution d'au moins l'image de projection sur le plan de projection autre que la région d'intérêt soit reréglée progressivement à une valeur plus élevée après que le moyen de génération d'image de projection (50) a terminé le calcul des valeurs de pixel des petites zones, en lesquelles le plan de projection a été divisé.

2. Appareil de génération d'image de projection selon la revendication 1, **caractérisé en ce que** le moyen de génération d'image de projection (50) calcule la valeur de pixel de chacune des petites zones à partir des valeurs de voxel de voxels des données de volume tridimensionnelles (100), à travers lesquelles un rayon qui a traversé les petites zones respectives et se déplace dans la direction de projection passe.

3. Appareil de génération d'image de projection selon la revendication 1 ou 2, **caractérisé en ce que** le moyen de changement de résolution (60) génère de façon répétée les images de projection de telle sorte que la résolution réglée soit augmentée progressivement à une valeur plus élevée de la région d'intérêt vers l'extérieur du plan de projection.

4. Programme pour amener un ordinateur d'un appareil de génération d'image de projection pour projeter des données de volume tridimensionnelles (100) qui ont été obtenues en imageant un sujet de telle sorte que la direction de projection ou sa position de projection puisse être changée par une opération depuis l'extérieur de l'appareil à fonctionner comme :
un moyen de réglage de résolution (40) pour régler la résolution d'une image de projection dans une région d'intérêt sur un plan de projection, sur lequel les données de volume tridimensionnelles (100) sont projetées, supérieure à celle de l'image de projection sur le plan de projection autre que la région d'intérêt lorsque la direction de projection ou la position de projection a été changée par l'opération ;
un moyen de génération d'image de projection (50) pour générer une image de projection en divisant, sur la base des résolutions réglées, le plan de projection en de petites zones qui ont des tailles différentes en fonction de si une zone du plan de projection à diviser est dans la région d'intérêt ou non et en calculant la valeur de pixel de chacune des petites zones divisées à partir des valeurs de voxel des données de volume tridimensionnelles (100) qui sont projetées sur les petites zones respectives ; et
un moyen de changement de résolution (60) **caractérisé en ce que** ledit moyen de changement de résolution est adapté pour générer de façon répétée des images de projection de telle sorte que la résolution d'au moins l'image de projection sur le plan de projection autre que la région d'intérêt soit reréglée progressivement à une valeur plus élevée après que le moyen de génération d'image de projection (50) a terminé le calcul des valeurs de pixel des petites zones, en lesquelles le plan de projection a été divisé.
